(19) Europäisches Patentamt
European Patent Office
Office européen des brevets

(11) Publication number: **0 147 155**
**A2**

(12) # EUROPEAN PATENT APPLICATION

(21) Application number: 84308833.7

(22) Date of filing: 17.12.84

(51) Int. Cl.⁴: C 07 J 1/00
C 07 J 51/00

(30) Priority: 22.12.83 US 564595

(43) Date of publication of application: 03.07.85 Bulletin 85/27

(84) Designated Contracting States: BE CH DE FR GB IT LI LU NL SE

(71) Applicant: ELI LILLY AND COMPANY
307, East McCarty Street
Indianapolis Indiana 46285(US)

(72) Inventor: Ward, John Stanley
241 E. Brunswick Avenue
Indianapolis Indiana 46227(US)

(72) Inventor: Jones, Charles David
223 E. Brunswick Avenue
Indianapolis Indiana 46227(US)

(74) Representative: Hudson, Christopher Mark et al,
Erl Wood Manor
Windlesham Surrey GU20 6PH(GB)

(54) **Improved method of preparing 2-Fluoro-17beta-estradiol.**

(57) 2-Fluoro-17β-estradiol is synthesized by mercurating at C-2 a 17β-estradiol diester or dietherate, displacing the mercury group with fluorine and then removing the ester- or ether-protecting groups.

EP 0 147 155 A2

Croydon Printing Company Ltd.

IMPROVED METHOD OF PREPARING 2-FLUORO-17β-ESTRADIOL

Liehr, Molecular Pharmacology, 23, 278 (1983) discloses the fact that, as contrasted with 17β-estradiol, 2-fluoro-17β-estradiol did not induce renal clear-cell carcinoma in male Syrian hamsters at equipotent estrogenic doses.

As a result, a renewed interest in 2-fluoro-17β-estradiol, first described by Utne et al., J. Org. Chem., 33, 2469 (1968), has occurred. The Utne procedure involves nitration of estrone in the 2-position and the 4-position, separation of the two isomers, conversion of the 3-phenolic hydroxyl of the 2-nitro estrone to an ether, reduction of the nitro group to an amine, diazotization of the amine including preparation of the fluoroborate salt, decomposition of the salt to yield 2-fluoroestrone 3-methyl ether, reduction of the 17-ketone group with sodium borohydride, followed by demethylation of the ether. This procedure involves many steps, the initial nitration produces a mixture of isomers, and the yields are not as high as would be desirable in a commercial process.

Neeman et al., J. Chem. Soc. (Perkin Transactions 1), 1972, 2300 used a different approach to the preparation of 2-fluoro-17β-estradiol. This approach uses as a starting material, 10β-hydroxyestr-4-ene-3,17-dione. This compound was epoxidized to yield the 4β,5β-epoxy derivative and the epoxy compound fluorinated with gaseous hydrogen fluoride in chloroform-ethanol to yield a 2-fluoro-10β-hydroxyestr-4-ene-3,17-dione. Dehydration of this compound with thionyl chloride produced 2-fluoroestrone, reduction of which with sodium borohydride yielded 2-fluoro-3,17β-estradiol.

The ultimate starting material for the Neeman synthesis, 19-nortestosterone, is not readily available. The authors also refer to their prior specific synthesis of 4-fluoro-17β-estradiol, a compound also prepared by Palmer et al., J. Labelled Compounds and Radiopharmaceuticals, 16, 14-16 (1979) and by Eakins et al., International Journal of Applied Radiation and Isotopes, 30, 695 (1979). These latter authors also prepared 4-fluoroestrone, 2,4-diiodoestradiol and 2-iodoestradiol. 2-Iodo-17β-estradiol was prepared from 17β-estradiol and sodium iodide. The preparation involved evaporation of any solvent and melting the two solid materials together at 180°C. Because radioactive iodine had been used, autoradiography could be used to distinguish the desired 2-iodo-17β-estradiol from all other substances in the mixture and separation by chromatography was possible. These radioactively labelled compounds, $I^{125}$-2-iodoestradiol and $F^{18}$-4-fluoroestradiol, were designed for use in scanning for estrogen dependent tumors or for scanning the prostate.

Heiman et al., J. Med. Chem., 23, 994 (1980) sought superior methods of preparing halogenated estrogens for use as estrogen receptor-based imaging agents. The authors prepared 3-fluorohexestrol by procedures similar to those employed by Utne et al.

Ng et al., J. Org. Chem., 46, 2520 (1981), describes the fluorination of hexestrol (a phenolic

estrogen), ortho to the phenol group, by converting an amine group to an arylazide followed by decomposing the azide in the presence of HF. In this last step, fluorine replaces the azide grouping.

Santaniello et al., J.C.S. Chem. Comm., 217, (1981) attempted to prepare orthohalogen derivatives of estradiol or of the corresponding ethers or acyl compounds. The authors regioselectively chloromercurated the $C_2$-position of 3-methoxy-17β-acetoxyestra-1,3,5-triene. This chloromercury derivative was readily converted to a 2-bromo and a 2-iodo derivative with $Br_2$ or $I_2$ respectively. An expansion of the original paper can be found at J.C.S. Chem. Comm., 1157 (1982).

Njar et al., J. Org. Chem., 48, 1007 (1983) describes the synthesis of 4-fluoro-17β-estradiol, 4-fluoroestrone and similar compounds. 4-Fluoro-17β-estradiol was prepared from 4-fluoro-19-nortestosterone by dehydrogenation with selenium dioxide.

A new reagent, acetyl hypofluorite, has been described by Rozen et al., J.C.S. Chem. Comm., 443 (1981) as a useful electrophilic fluorinating agent.

Shiue et al., J. Nucl. Med., 23, 899 (1982) used $^{18}F$-labeled acetyl hypofluorite to fluorinate 2-deoxy-D-glucose.

Mantescu et al., Radiopharm. and Label. Cmpds., 395 (1973) employed a mixture of glacial acetic acid and $K^{18}F$ as a fluorinating agent to prepare certain steroid hormones containing radioactive fluorine. These hormones included 4-fluorotestosterone and 2,4-difluoroestrone. The authors adopted the procedure of iodinating estrone at the 2 and 4 carbons and then replacing the iodine with radiofluorine using the KF-acetic anhydride procedure.

At present, however, no commercially feasible synthetic route is available for preparing 2-fluoro-17β-estradiol; such a procedure must use readily available and inexpensive starting materials, employ a minimal number of steps and be capable of giving good yields. This invention provides such a procedure.

In accordance with the invention, there is provided a process for preparing a 2-fluoroestradiol of Formula (1):

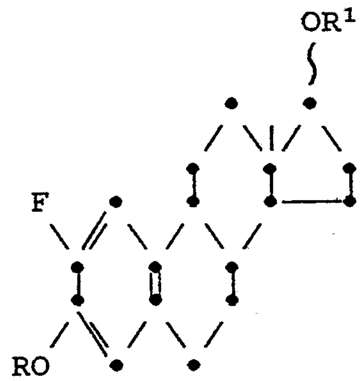

(1)

in which R and $R^1$ are hydroxy-protecting groups or hydrogen, which comprises:

a) reacting with acetyl hypofluorite a compound of Formula (2):

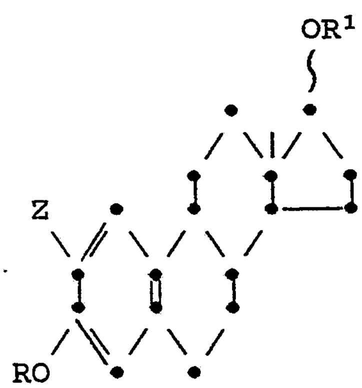

(2)

, in which

Z is a mercuric moiety and R and $R^1$ are as defined above; and

b) if desired, removing any protecting group present in the product.

The reaction scheme is illustrated in the following flow chart. Mercury trifluoroacetate in trifluoroacetic acid is used as the mercurating agent for illustrative purposes only.

FLOW CHART

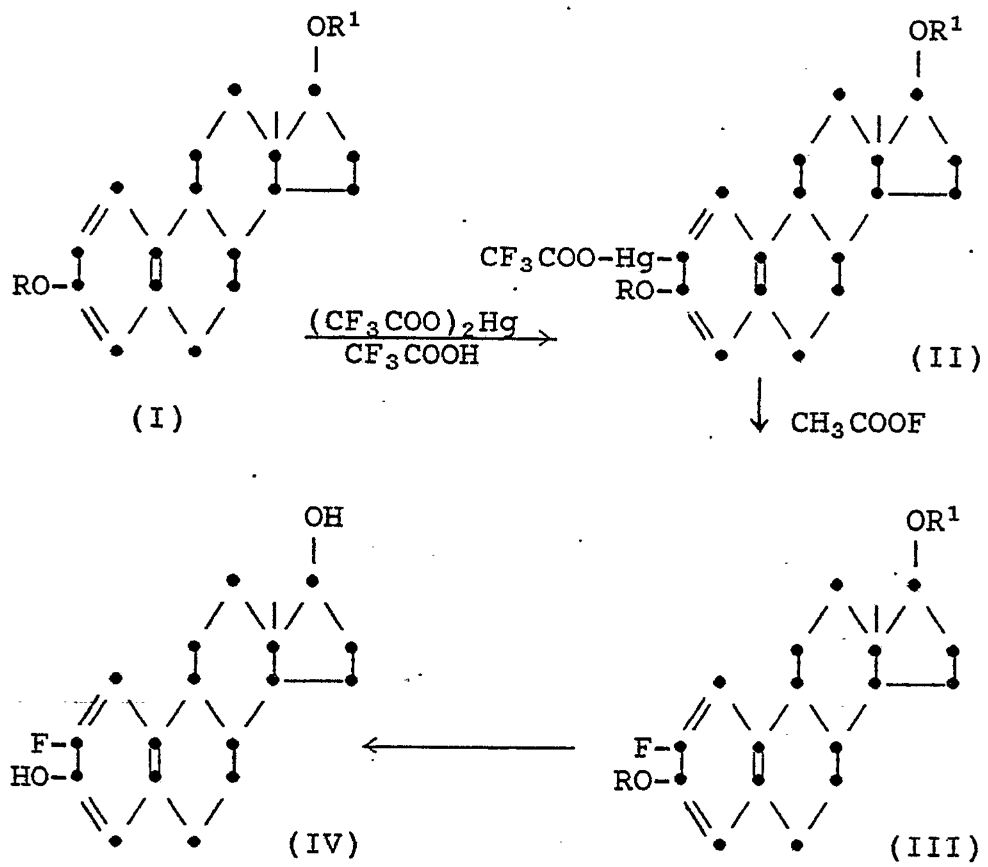

R and $R^1$ are, independently, hydroxy-protecting groups. The term "hydroxy-protecting group" means a protecting group which is stable under the reaction conditions of the present process. Such protecting groups will be familiar to those skilled in the art. See for example, Protecting Groups in Organic Chemistry, McOmie, Ed., Plenum Press, N.Y. (1973); and Protective Groups in Organic Synthesis, Greene, Ed., John Wiley & Sons, N.Y. (1981).

In the above procedure, the preferred hydroxy-protecting group (one stable to acidic solvents such as $CF_3COOH$ under the reaction conditions or to acid containing reaction media) can be an ether such as a lower alkyl ether or an ester such as a formate, lower-alkanoate, fluoro-substituted-lower alkanoate, benzoate, lower alkyl sulfonate or aryl sulfonate. Acid-stable, lower-alkyl ether protecting groups may include, among others, methyl, ethyl, isopropyl and n-propyl ethers. Lower-alkanoyl or fluoro-substituted lower-alkanoyl protecting groups may include groups such as acetate, propionate, isobutyrate, n-butyrate, and trifluoroacetate esters. Finally, lower-alkylsulfonyl protecting groups may include methanesulfonate (mesylate), trifluoromethane sulfonate, ethanesulfonate and the like, while aryl sulfonate protecting groups may include benzenesulfonate, tosylate and the like.

In the above flow chart, the mercurating agent has been specified as mercuric trifluoroacetate and the solvent as trifluoroacetic acid or acetic acid. Other mercurating agents such as mercuric acetate, mercuric

nitrate, or mercuric fluoride, in trifluoroacetic acid or acetic acid, also can be used. Thus, as used herein, "mercuric moiety" refers to groups such as $-Hg(OCOCF_3)$, -Hg(OAc), $-HgNO_2$, -HgCl, and -HgF.

The extent and selectivity of the mercuration, C-2 versus C-4, depends upon the nature of the protecting group. Using an acetate protecting group (R and $R^1$ are $CH_3$-CO in Formulae (I), (II) and (III)), mercuration occurs nearly exclusively at C-2; hence, the synthetic procedure yields a 2-mercurated derivative which can be transformed to 2-fluoro-17β-estradiol essentially free from any 4-fluoro products. With other protecting groups, some C-4 mercuration may occur. Separation of the 4-mercurated 17β-estradiols from 2-mercurated 17β-estradiols by chromatography provides 2-fluoro-17β-estradiol free from products carrying a 4-fluoro group. Alternatively, any 4-fluoro compound can be removed at either the 17β-diprotected stage of the synthetic procedure (III) or as final products (IV). Using the 3,17β-diacetate as a starting material is preferred because production of isomeric products is minimal.

In addition to the choice of protecting groups used, to prepare substantially pure 2-fluoro-17β-estradiol, the solvent and reagents should be free from other halogens which might exchange with F under the reaction conditions. Presence of chloride, for example, in the reaction medium or as part of the mercury-containing group at C-2 might result in co-production of 2-chloro-17β-estradiol.

In the acetyl hypofluorite reaction, trifluoroacetic acid has been specified as the mutual inert solvent, but it will be apparent to those skilled in the art that other equivalent acidic solvents could be employed. It should be emphasized, however, that the solvent, as well as the acetyl hypofluorite reagent itself should be substantially free of other exchangeable halogens to avoid the inadvertent production of other 2-halo-17β-estradiols.

Finally, the protecting groups, R and $R^1$, must be removed from product (III) in order to obtain the final product, 2-fluoro-17β-estradiol (IV). If the protecting group is an ester group, base hydrolysis usually is usually employed, using an alkali metal hydroxide or carbonate as the base. Reduction with $LiAlH_4$ also can be used as can trans-esterification using an alcohol like methanol and a acidic catalyst. The methyl ether protecting groups can be cleaved with such reagents as pyridine hydrochloride or $AlBr_3$ in the presence of ethyl mercaptan or by other established ether cleavage methods. Methyl ether cleavage procedures are well-known in the art. Hence, the methyl ether is the preferred ether protecting group. However, those skilled in the art readily could adapt procedures for cleaving a methyl ether to cleavage of higher alkyl ethers. For more detailed information as to the use and cleavage of hydroxy protecting groups, see Greene, Protecting Groups in Organic Synthesis, Chapters 2 and 3 (John Wiley & Sons, N.Y. (1981)).

When R and R' are both acetyl, up to 30% yields of 2-fluoro-17β-estradiol diacetate, substantially free from contaminating 4-fluoro products, are obtained. Hydrolysis of the two acetate groups is virtually quantitative (95%). Thus, the use of the diacetate of 17β-estradiol as a starting material is preferred.

The invention is further illustrated by the following non-limiting examples.

## Example 1

Preparation of 3,17β-estradiol diacetate

Although 3,17β-estradiol diacetate is commercially available, as is 17β-estradiol, we prepared the diacetate from 3,17β-estradiol according to the following procedure.

A reaction mixture was prepared from 10.8 g. of 3,17β-estradiol, 40 ml. of acetic anhydride and 15 g. of polymeric polyvinyl pyridine in 200 ml. of anhydrous chloroform. The reaction mixture, a suspension, was refluxed overnight. The volatile constitutents were removed at about 40°C. The resulting residue was dissolved in a mixture of from 1200-1500 ml. of ethyl acetate and 500 ml. of water. This new mixture was stirred for about 15-20 minutes at ambient temperature after which time the polymer was separated by filtration. The filtered polymer was rinsed with additional ethyl acetate. The ethyl acetate filtrate and wash were combined and the combined layer washed twice with water. The water layer was also extracted with ethyl acetate and this ethyl acetate extract added to the ethyl acetate filtrate. The combined ethyl acetate fractions were given a final water wash and were then dried. Evaporation of the ethyl acetate in vacuo yielded an oil which crystallized upon scratching. Yields of 3,17β-estradiol 3,17β-diacetate in two runs of the above quantities gave 13 and 13.1 g. of the desired product (yield = 92-93%). 3,17β-Estradiol diacetate has a melting point of 125°-126°C.

## Example 2

Preparation of 2-trifluoroacetoxymercury-3,17β-estradiol diacetate

About 10 g. of 3,17β-estradiol 3,17β-diacetate were dissolved in 15 ml. of trifluoroacetic acid and the solution cooled to about 0°C. Twelve grams of mercuric trifluoroacetate were added and the reaction mixture stirred at ice bath temperatures for about 3.5 hours. The solvent was then removed by evaporation in vacuo and the residual material dissolved in methylene dichloride. The methylene dichloride solution was extracted with water and the water extract discarded. The methylene dichloride layer was dried over anhydrous sodium sulfate, filtered and the methylene dichloride evaporated from the filtrate in vacuo. The resulting residual foam was triturated with hexane by sonication

for about one hour to produce crystals. The crystallization mixture was filtered and the filter cake rinsed with hexane. About 17.2 g. of the 2-trifluoroacetyl mercury derivative were obtained (92% yield); m.p. 122°C (with decomposition).

NMR ($CDCl_3$): δ 0.82 (s, 3H 18-$CH_3$), δ 2.04 (s, 3H, acetyl), δ 2.32 (s, 3H, acetyl), δ 4.66 (t, 1H, 17 α-H), δ 6.88 (s, 1H, 4-H) δ 7.14 (s, 1H, 1-H).

## Example 3

Preparation of 2-fluoro-3,17β-estradiol diacetate

Acetyl hypofluorite reagent was prepared as follows:

A suspension of 5 g. of sodium acetate in 50 ml. of glacial acetic acid was added to 550 ml. of Freon 11 with stirring. The mixture was cooled to about -80° C. in a dry ice-acetone bath while nitrogen was being bubbled through the reaction mixture. When the reaction mixture had attained the desired temperature, a stream of 18% fluorine in nitrogen was bubbled in. After five to six hours, a 2 ml. aliquot was added to 20 ml. of 60% aqueous acetic acid containing 1.5 g. of potassium iodide. The iodine generated was titrated with sodium thiosulfate and indicated that the solution was approximately 0.145 molar in acetyl hypofluorite.

Two grams of 2-trifluoroacetylmercury-17β-estradiol diacetate were dissolved in 25 ml. of chloroform. To this stirred solution was added 25 ml. of the 0.145 molar acetyl hypofluorite mixture prepared as described above. After about 10 minutes, the mixture was combined with other similar reaction mixtures. The combined reaction mixtures were then washed with water, with aqueous saturated sodium bicarbonate and again with water. The extracts were then dried. Evaporation of the solvent in vacuo yielded a residual yellowish oil.

Thirty-eight similar runs employing a total of 76 g. of the 2-trifluoroacetylmercury-17β-estradiol diacetate starting material were combined and the combined residues obtained after workup, were chromatographed on an HPLC over a silica gel column using a 10% ethyl acetate -- 90% isooctane solvent mixture as the eluant. Those fractions containing 2-fluoro-17β-estradiol diacetate analysis were combined to give 9.5 g. of the desired compound. This material was dissolved in toluene and rechromatographed over a HPLC silica gel column with toluene as the eluant. The desired 2-fluoro-17β-estradiol diacetate (7.7 g.) was obtained. m.p. 122-124°C.

NMR ($CDCl_3$): δ 0.84 (s, 3H, 18-$CH_3$), δ 2.02 (s, 3H, acetyl), δ 2.30 (s, 3H, acetyl), δ 4.66 (t, 1H, 17α-H), δ 6.78 (s, 1H, $J_{H-F}$= 6Hz, 4-H), δ 7.04 (d, 1H, $J_{H-F}$= 13.5Hz, 1-H).

## Example 4

### Preparation of 2-fluoro-17β-estradiol

2-Fluoro-17β-estradiol diacetate (3.6 g.) was suspended in 48 ml. of methanol and 15 ml. of water. A solution of 2.4 g. of potassium hydroxide in 29 ml. of water was added. The reaction mixture was stirred overnight at ambient temperature. The volatile constituents were removed in vacuo and the resulting residue suspended in 1N aqueous hydrochloric acid. This suspension was slurried with ethyl acetate until two clean layers formed. The aqueous layer was separated and the separated layer washed with additional ethyl acetate. The ethyl acetate extracts were combined, washed twice with water and then dried over $Na_2SO_4$. The combined solutions were concentrated to less than 50 ml. and crystallization induced by scratching. The crystallizing solution was placed at about 0°C. overnight. The crystals produced were separated by filtration and the filter cake washed with pre-cooled methanol, producing 2.9 g. of 2-fluoro-17β-estradiol (75% yield). The compound crystallized with 1/2 mole of methanol. m.p. 168-169°C.

NMR ($CDCl_3$) δ 0.66 (s, 3H, 18-$CH_3$), δ 4.44 (d, 1H, J=5Hz, 17α-H), δ 6.54 (d, 1H, $J_{H-F}$= 9Hz, 4-H) δ 6.88 (d, 1H, $J_{H-F}$= 13Hz, 1-H).

Analysis for the hemimethanolate of 2-fluoro-17β-estradiol was as follows:

Calculated: C, 72.51; H, 8.22
Found: C, 72.30; H, 8.49

The above procedure is equally adaptable to the preparation of 2-fluoro-17α-estradiol.

In addition to providing 2-fluoro-17β-estradiol as an estrogenic substance, the above procedure may be used to produce 2-$^{18}$F-17β-estradiol useful in position emission tomography in estrogen dependent tumors, etc.

As previously stated, 2-fluoro-17β-estradiol has a lower cancer-inducing potential than 17β-estradiol. In addition, 2-fluoro-17β-estradiol is metabolized more slowly than 17β-estradiol and thus lower dose levels may be used to provide equal estrogenic activity.

CLAIMS

1. A process for preparing a 2-fluoroestradiol of Formula (1):

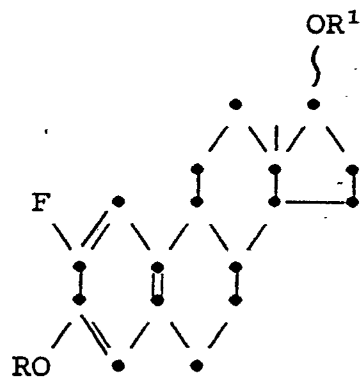

(1)

in which R and $R^1$ are hydroxy-protecting groups or hydrogen, which comprises:

a) reacting with acetyl hypofluorite a compound of Formula (2):

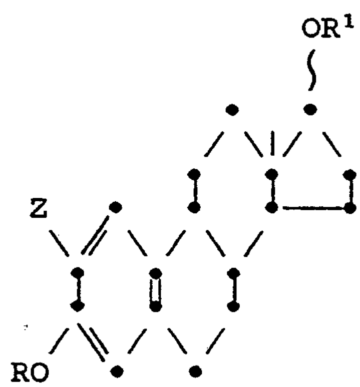

(2)

, in which

Z is a mercuric moiety and R and $R^1$ are as defined above; and

b) if desired, removing any protecting group present in the product.

2. A process as claimed in claim 1, in which -OR or $-OR^1$, independently, is a lower alkyl ester or ether.

3. A process as claimed in claim 2 in which R and $R^1$ both are acetyl.

4. A process as claimed in any one of claims 1 to 3 in which any solvent used is free of other halogens exchangeable with fluorine.

5. A process as claimed in claim 4 in which the solvent is trifluoroacetic acid.

6. A process as claimed in claim 5 in which Z is $CF_3COOHg-$.

7. A process as claimed in claim 6 in which 2-fluoro-17β-estradiol is produced.

8. A 2-fluoroestradiol of Formula (1) whenever prepared according to a process as claimed in claim 1.

9. A compound of Formula (2), as defined in claim 1, in which Z is $-Hg(OCOCF_3)$.